# EUROPEAN PATENT SPECIFICATION

(11) **EP 4 637 535 B1**
(45) Date of publication and mention of the grant of the patent: **21.10.2026**
(21) Application number: 24709887.4
(22) Date of filing: 11.01.2024
(51) Int. Cl.: A61B 5/0225

(54) **ARTERIAL PRESSURE CALIBRATION**
ARTERIENDRUCKKALIBRIERUNG
ÉTALONNAGE DE PRESSION ARTÉRIELLE

(30) Priority: 12.01.2023 US 202363479717 P
(43) Date of publication of application: 29.10.2025
(73) Proprietor: Becton, Dickinson and Company, Franklin Lakes, NJ 07417-1880 (US)
(72) Inventor: KUIJKENS, Hans, Jean Paul, 1101 BE Amsterdam (NL); AL HATIB, Feras, Irvine, California 92614 (US); VAN GOUDOEVER, Jeroen, 1101 BE Amsterdam (NL); REUDERINK, Boris, 3818 BG Amersfoort (NL)
(74) Representative: Venner, Julia Ann
(86) International application number: PCT/US2024/011197
(87) International publication number: WO 2024/151829

(56) References cited:
- WO-A1-2020/112555
- US-A1- 2018 070 837
- KAREL H WESSELING ET AL: "Physiocal, calibrating finger vascular physiology for Finapres", HOMEOSTASIS, vol. 36, no. 2-3, 1 January 1995 (1995-01-01), pages 67 - 82, XP055210776

## Description

### CROSS-REFERENCE TO RELATED APPLICATION(S)

This application claims the benefit of U.S. Provisional Application No. 63/479,717, filed January 12, 2023, and entitled "ARTERIAL PRESSURE CALIBRATION".

### BACKGROUND

The present disclosure relates to arterial pressure sensing, and more particularly to calibration factors for arterial pressure measurements made using a volume clamping technique.

Some non-invasive arterial pressure sensors generate a pressure reading by clamping (i.e., holding constant) arterial volume using plethysmographic readings. A volume setpoint and a fast servo system are used to make arterial pressure waveform measurements using a volume clamp technique. However, it can be difficult to acquire the proper setpoint, which can lead to inaccurate arterial pressure waveform data.

US 2018/070837 describes an apparatus for measuring the blood pressure, BP, of a user, the apparatus comprising a volume-clamp BP monitoring device that comprises a first pressure device for applying pressure to a first part of the body of the user, a first photoplethysmogram, PPG, sensor for obtaining a first PPG signal from the first part of the body of the user, and a control unit that is configured to analyse the first PPG signal and to control the pressure of the first pressure device; wherein the control unit is configured to adjust the pressure of the first pressure device to maintain the first PPG signal at a constant level and to determine the BP of the user from the pressure of the first pressure device; and a second sensor, separate from the first PPG sensor, for measuring a physiological characteristic of the user in a second part of the body of the user, wherein the second part of the body is separate from the first part of the body; wherein the apparatus is configured to analyse the measured physiological characteristic to determine a measure of the blood perfusion in the second part of the body of the user, and to determine whether to perform a recalibration of the volume-clamp BP monitoring device on the basis of changes in the blood perfusion.

### SUMMARY

An example of a method of measuring arterial pressure includes continuously varying an air pressure of an annular air bladder to maintain a constant volume based arterial volume data from a plethysmographic sensor, receiving a first air pressure signal representative of the air pressure of the air bladder while the air pressure is continuously varied, and determining a first mean arterial pressure value. The air pressure of the annular air bladder is continuously varied by an air pressure controller during a first time period, the first air pressure signal is received from the air pressure controller, and the first mean arterial pressure value is determined by a processor. The method further includes adjusting an air pressure of the annular air bladder from a first air pressure to a second air pressure and receiving a plurality of arterial volume signals from the plethysmographic sensor as the air pressure is adjusted from the first air pressure to the second air pressure. The air pressure is adjusted from the first pressure to the second pressure by the air pressure controller during a second time period. The plurality of arterial volume signals is received by the processor and is representative of a plurality of arterial volume waveforms. Further, each arterial volume waveform of the plurality of arterial volume waveforms corresponds to an air pressure between the first air pressure and the second air pressure. The method further comprises determining a second mean arterial pressure value based on the plurality of arterial volume waveforms and generating a calibration factor. The calibration factor is based on the second mean arterial pressure value and the first mean arterial pressure value, and is applied to arterial pressure waveform data to compensate for variations in operation of the air bladder. The second mean arterial pressure value is determined by the processor and the calibration factor is generated by the processor.

An example of a system for measuring arterial pressure includes a plethysmographic sensor configured to sense arterial volume, an air pressure controller pneumatically connected to an annular air bladder and configured to adjust an air pressure of the annular airbladder, a processor in operable communication with the air pressure controller and the plethysmographic sensor, and memory encoding executable instructions. The instructions, when executed, cause the processor to, via the air pressure controller, continuously vary an air pressure of the annular air bladder to maintain a constant volume based arterial volume data from the plethysmographic sensor, receive a first air pressure signal representative of the air pressure of the air bladder while the air pressure is continuously varied, and determine a first mean arterial pressure value. The air pressure of the annular air bladder is continuously varied a first time period and the first air pressure signal is received from the air pressure controller. The first air pressure signal representative of a first arterial pressure waveform and the first mean arterial pressure value is determined based on the first arterial pressure waveform. The instructions, when executed, further cause the processor to cause the air pressure controller to adjust the air pressure of the annular air bladder from a first air pressure to a second air pressure and receive a plurality of arterial volume signals from the plethysmographic sensor as the air pressure is adjusted from the first air pressure to the air second pressure. The air pressure is adjusted from the first pressure to the second pressure by the air pressure controller during a second time period. The plurality of arterial volume signals is representative of a plurality of arterial volume waveforms and each arterial volume waveform of the plurality of arterial volume waveforms corresponds to an air pressure between the first air pressure and the second air pressure. The instructions, when executed, further cause the processor to determine a second mean arterial pressure value based on the plurality of arterial volume waveforms and generate a calibration factor. The calibration factor is based on the second mean arterial pressure value and the first mean arterial pressure value, and is applied to arterial pressure waveform data to compensate for variations in operation of the air bladder.

An example of a method of measuring arterial pressure includes receiving a first mean arterial pressure value, adjusting an air pressure of an annular air bladder from a first air pressure to a second air pressure and receiving a plurality of arterial volume signals from the plethysmographic sensor as the air pressure is adjusted from the first air pressure to the second air pressure. The first mean arterial pressure value is received by a processor and the air pressure is adjusted from the first pressure to the second pressure by an air pressure controller. The plurality of arterial volume signals is received by the processor and is representative of a plurality of arterial volume waveforms. Further, each arterial volume waveform of the plurality of arterial volume waveforms corresponds to an air pressure between the first air pressure and the second air pressure. The method further comprises determining a second mean arterial pressure value based on the plurality of arterial volume waveforms and generating a calibration factor. The calibration factor is based on the second mean arterial pressure value and the first mean arterial pressure value, and can be applied to arterial pressure waveform data to compensate for variations in operation of the air bladder. The second mean arterial pressure value is determined by the processor and the calibration factor is generated by the processor.

The present summary is provided only by way of example, and not limitation. Other aspects of the present disclosure will be appreciated in view of the entirety of the present disclosure, including the entire text, claims, and accompanying figures.

### BRIEF DESCRIPTION OF THE DRAWINGS

FIG. 1 is a schematic diagram of an example of a system for sensing arterial pressure.
FIG. 2 is an isometric view of an example of a non-invasive sensor suitable for use with the system of FIG. 1.
FIG. 3 is a flow diagram illustrating an example of a method of generating an arterial pressure calibration factor suitable for use with the systems of FIGS. 1 and 2.
FIG. 4 is a flow diagram illustrating an example of a method of generating adjusted arterial pressure information suitable for use with the systems of FIGS. 1 and 2 and also with the method of FIG. 3.
FIG. 5A is a scatter plot comparing arterial pressure data generated using catheter- and clamping-based techniques.
FIG. 5B is a scatter plot comparing the catheter-based arterial pressure data of FIG. 5A with the clamping-based arterial pressure data of FIG. 5A adjusted according to the method of FIG. 4.

While the above-identified figures set forth one or more examples of the present disclosure, other examples are also contemplated, as noted in the discussion. In all cases, this disclosure presents the invention by way of representation and not limitation. It should be understood that numerous other modifications and examples can be devised by those skilled in the art, which fall within the scope and spirit of the principles of the invention. The figures may not be drawn to scale, and applications and examples of the present invention may include features and components not specifically shown in the drawings.

### DETAILED DESCRIPTION

The present disclosure describes an approach for creating calibration factors for calibrating arterial pressure measurements. More specifically, the present disclosure describes systems and methods for creating and using calibration factors to calibrate arterial pressure measurements of a volume-clamped arterial volume. The calibration factors disclosed herein are created by using a ratio of mean arterial pressure ("MAP") values. The MAP values used for the calibration factors can be created based on data collected by a single sensor and are based on signal analysis of both arterial pressure data and arterial volume data.

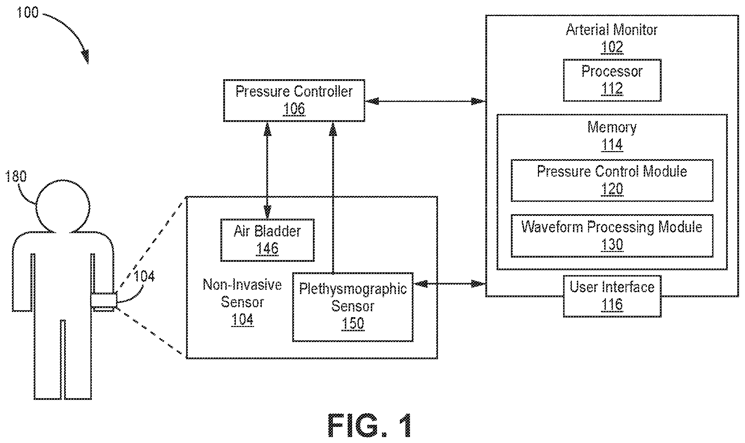

FIG. 1 is a schematic diagram of hemodynamic sensing system 100, which is an example of a system for sensing and using hemodynamic data. System 100 includes arterial monitor 102, non-invasive sensor 104, and air pressure controller 106. Arterial monitor 102 includes processor 112, memory 114, and user interface 116. Memory 114 stores pressure control module 120 and waveform processing module 130. Non-invasive sensor 104 includes air bladder 146 and plethysmographic sensor 150. FIG. 1 also depicts patient 180, who is shown as wearing non-invasive sensor 104. Hemodynamic sensing system 100 is configured to perform one or more methods described herein. Hemodynamic sensing system 100 is configured to sense and use hemodynamic data, such as arterial volume data and/or arterial pressure data. More generally, hemodynamic sensing system 100 is configured to perform any of the functions attributed herein to a hemodynamic sensor or a hemodynamic sensing system, including receiving an output from any source referenced herein, detecting any condition or event referenced herein, and generating and providing data and information as referenced herein.

Processor 112 can execute software, applications, and/or programs stored on memory 114. Examples of processor 112 can include one or more of a processor, a microprocessor, a controller, a digital signal processor (DSP), an application specific integrated circuit (ASIC), a field-programmable gate array (FPGA), or other equivalent discrete or integrated logic circuitry. Processor 112 can be entirely or partially mounted on one or more circuit boards.

Memory 114 is configured to store information and, in some examples, can be described as a computer-readable storage medium. Memory 114, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). In some examples, memory 114 is a temporary memory. As used herein, a temporary memory refers to a memory having a primary purpose that is not long-term storage. Memory 114, in some examples, is described as volatile memory. As used herein, a volatile memory refers to a memory that the memory does not maintain stored contents when power to the memory 114 is turned off. Examples of volatile memories can include random access memories (RAM), dynamic random access memories (DRAM), static random access memories (SRAM), and other forms of volatile memories. In some examples, the memory is used to store program instructions for execution by the processor. The memory, in one example, is used by software or applications running on hemodynamic sensing system 100 (e.g., by a computer-implemented machine learning model or a data processing module) to temporarily store information during program execution.

Memory 114, in some examples, also includes one or more computer-readable storage media. Memory 114 can be configured to store larger amounts of information than volatile memory. Memory 114 can further be configured for long-term storage of information. In some examples, memory 114 includes non-volatile storage elements. Examples of such non-volatile storage elements can include, for example, magnetic hard discs, optical discs, floppy discs, flash memories, or forms of electrically programmable memories (EPROM) or electrically erasable and programmable (EEPROM) memories.

User interface 116 is an input and/or output device and enables an operator to control operation of hemodynamic sensing system 100. For example, user interface 116 can be configured to receive inputs from an operator and/or provide outputs. User interface 116 can include one or more of a sound card, a video graphics card, a speaker, a display device (such as a liquid crystal display (LCD), a light emitting diode (LED) display, an organic light emitting diode (OLED) display, etc.), a touchscreen, a keyboard, a mouse, a joystick, or other type of device for facilitating input and/or output of information in a form understandable to users and/or machines.

Non-invasive sensor 104 is a wearable and non-invasive sensor for sensing hemodynamic parameters. More specifically, non-invasive sensor 104 is able to sense hemodynamic parameters using air bladder 146 and plethysmographic sensor 150, and does not require physically-invasive techniques for operation. Non-invasive sensor 104 is configured to sense both arterial pressure waveforms and arterial volume waveforms using a combination of air bladder 146 and plethysmographic sensor 150, as will be explained in more detail subsequently.

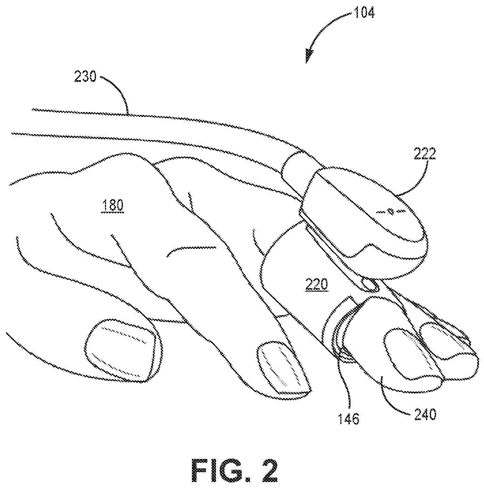

Air bladder 146 is an annular and pressurizable bladder capable of applying variable mechanical pressure to an appendage of patient 180. As will be explained in more detail subsequently, the pressure of air bladder 146 can be adjusted by air pressure controller 106. Air bladder 146 is deformable such that as the air pressure of air bladder 146 is adjusted, air bladder 146 expands and exerts force on the appendage of patient 180, constricting arterial volume in the appendage. The portion of the appendage of patient 180 surrounded circumferentially by air bladder 146 defines a clamped volume when air bladder 146 applies or exerts a force on the appendage. Air bladder 146 is generally repositionable on appendages of patient 180. The force exerted by air bladder 146 can be referred to as a "clamping force" in some examples. As will be described subsequently with respect to FIG. 2, air bladder 146 can be formed as an annular cuff that can be disposed circumferentially around a finger of patient 180, such that air bladder 146 circumferentially surrounds the finger of patient 180 when worn and can apply pressure to one or more arteries of the appendage located circumferentially within the annular cuff. As air is flowed into the air bladder 146, the annular air bladder 146 can expand and exert force on the finger and restrict arterial volume within the finger.

Plethysmographic sensor 150 is configured to sense arterial volume of arteries of patient 180. Specifically, plethysmographic sensor 150 is configured to sense arterial volume within the clamped volume defined by the structure and position of air bladder 146. Arteries of patient 180 pulsate during normal blood flow, expanding (with systolic pressure) and relaxing (with diastolic pressure) in volume over the course of each heartbeat cycle. Plethysmographic sensor 150 can sense the expansion and contraction of arteries within the clamped volume during arterial pulsation. Plethysmographic sensor 150 is electronically connected to processor 112, such that in operation processor 112 can control the operation of plethysmographic sensor 150 and/or plethysmographic sensor 150 can provide plethysmographic signals to processor 112 representative of arterial volume.

In some examples, plethysmographic sensor 150 can be a photoplethysmographic sensor including a light emitter and a light sensor. The light emitter is structured and configured to emit light through arteries of patient 180 and the light sensor is configured to receive light emitted by the light emitter after the light has passed through arteries of patient 180. The relative intensity of the received light signal is inversely proportional to arterial volume, such that reduced signal corresponds to increased arterial volume and increased signal corresponds to decreased arterial volume.

Air pressure controller 106 is pneumatically connected to air bladder 146 and is configured to adjust the air pressure of air bladder 146. Air pressure controller 106 can include one or more valves, pumps, pressurized air sources, and/or other pneumatic components capable of selectively causing air to flow to air bladder 146 or out of air bladder 146 to adjust the air pressure inside of air bladder 146. Air pressure controller 106 also includes one or more pressure sensors for sensing the pressure of air within air bladder 146 as well as one or more electronic components for receiving signals from and sending signals to processor 112, such as one or more electronic circuits or controllers. For example, the electronic components of air pressure controller 106 can be configured to receive control signals from processor 112 and/or to send pressure readings from the pressure sensor to processor 112.

Air pressure controller 106 can be operated to cause pressurized air to flow into air bladder 146 in order to increase the air pressure within air bladder 146 and/or to allow air to flow out of air bladder 146 to decrease the air pressure within air bladder 146. For example, air pressure controller 106 can control the position of one or more valves disposed between a source of pressurized air and air bladder 146 to selectively allow air to flow from the pressurized air source to the interior of air bladder 146. Air pressure controller 106 can also be structured and/or configured to control the position of a vent valve to selectively allow air to flow out of air bladder 146. As a further example, air pressure controller 106 can include one or more pumps for compressing air that can be flowed to the interior of air bladder 146. As described previously, the air bladder 146 is deformable such that the air pressure within air bladder 146 can be varied to adjust the force exerted by air bladder 146 on the appendage circumferentially-surrounded by air bladder 146.

Arterial monitor 102 is configured to control operation of air pressure controller 106, such that arterial monitor 102 can cause air pressure controller 106 to adjust the air pressure of air bladder 146. As depicted in FIG. 1, memory 114 includes pressure control module 120. Pressure control module 120 includes one or more executable programs that, when executed, cause processor 112 to cause air pressure controller 106 to adjust the air pressure of air bladder 146. The programs of pressure control module 120 can allow for the air pressure of air bladder 146 to be adjusted to a particular pressure value and/or according to one or more patterns. The patterns can include, for example, a continuous or segmented pressure ramp, as will be discussed in more detail subsequently.

Air pressure controller 106 and plethysmographic sensor 150 are electronically-connected to arterial monitor 102, allowing processor 112 to send signals to and receive signals from both air pressure controller 106 and plethysmographic sensor 150. As will be explained in more detail, air pressure controller 106 can be configured to adjust the pressure of air bladder 146 based on the plethysmographic signals from plethysmographic sensor 150. Processor 112 can receive plethysmographic signals from plethysmographic sensor 150 and cause air pressure controller 106 to adjust the air pressure of air bladder 146 according to the received signals. Processor 112 can also adjust receive pressure signals from air pressure controller 106 describing the air pressure of air bladder 146.

Memory 114 also includes waveform processing module 130. Waveform processing module 130 includes one or more executable programs that can be executed by processor 112 to analyze pressure waveforms received from air pressure controller 106 and/or plethysmographic waveforms received from plethysmographic sensor 150. Processor 112 can use outputs of the programs of waveform processing module 130 to create calibration factors according to methods disclosed herein, as will be described in more detail subsequently.

In operation, non-invasive sensor 104 can be used to measure the arterial pressure of patient 180 by combined operation of plethysmographic sensor 150 and air pressure controller 106. Specifically, processor 112 can control operation of air pressure controller 106 according to received arterial volume data from plethysmographic sensor 150 and continuously adjust the air pressure within air bladder 146 to mechanically oppose arterial volume changes within the clamped volume, thereby allowing air pressure controller 106 to cause the arterial volume of arteries within the clamped volume of air bladder 146 to remain constant or substantially constant. For example, air pressure controller 106 can include a proportional-integral-derivative (PID) controller that can be used to control the pressure of air bladder 146 according to the signal from plethysmographic sensor 150. Air pressure controller 106 can receive plethysmographic signals directly from plethysmographic sensor 150 in these examples. Additionally and/or alternatively, pressure control module 120 of memory 114 can include one or more programs that enables processor 112 function as a PID controller and cause air pressure controller 106 to adjust the air pressure of air bladder 146 to maintain a constant or near constant arterial volume signal from plethysmographic sensor 150. The pressure required to maintain a constant or near arterial volume of arteries within air bladder 146 represents the arterial pressure of patient 180, and air pressure controller 106 can provide that pressure signal to arterial monitor 102. The pressure signal can be processed by processor 112 and one or more programs of memory 114, and/or can be presented to a user by user interface 116. An arterial pressure measurement using air pressure controller 106 and plethysmographic sensor 150 as described herein can be referred to as a "volume clamp" measurement.

Non-invasive sensor 104 can also be used to sense arterial volume fluctuations during blood flow. Processor 112 can execute programs of pressure control module 120 to cause air pressure controller 106 to hold the air pressure of air bladder 146 at a constant value. Processor 112 can receive plethysmographic signals representative of arterial volume from plethysmographic sensor 150 while air bladder 146 is held at the constant volume, and can analyze the signals received with one or more programs of waveform processing module 130.

In some examples, air pressure controller 106 can include one or more logic-capable hardware elements. For example, air pressure controller 106 can include a separate processor, memory, and/or user interface that are substantially similar to processor 112, memory 114, and/or user interface 116, respectively that are able to execute the programs of pressure control module 120 to cause air pressure controller 106 to adjust the air pressure of air bladder 146 to a desired pressure or according to a desired pattern. Further, while arterial monitor 102 and non-invasive sensor 104 are shown as separate elements in FIGS. 1-2, arterial monitor 102 and non-invasive sensor 104 can be formed as separate subcomponents or subsystems of a hemodynamic sensing system 100 capable of performing the methods described herein.

FIG. 2 is a detailed isometric view of non-invasive sensor 104 installed on an appendage of patient 180. FIG. 2 depicts non-invasive sensor 104, including air bladder 146, and patient 180. In FIG. 2, non-invasive sensor 104 also includes cuff housing 220, sensor housing 222, and control line 230. Patient 180 includes finger 240.

Cuff housing 220 is a rigid housing element that extends around air bladder 146, such that air bladder 146 is circumferentially surrounded by cuff housing 220. In the depicted example, cuff housing 220 is annular and air bladder 146 also adopts a generally annular shape. Cuff housing 220 is attached to sensor housing 222. Sensor housing 222 houses plethysmographic sensor 150 and, in some examples, sensor housing 222 can also house air pressure controller 106. Control line 230 includes electronic communication lines for enabling communication between plethysmographic sensor 150 and processor 112 as well as pneumatic channels for channel air to the interior of air bladder 146. In the depicted example, the appendage of patient 180 for which arterial pressure and arterial volume can be measured is finger 240. The clamped volume in the depicted example is the portion of finger 240 that is circumferentially surrounded by air bladder 146. In other examples, cuff housing 220, air bladder 146, and/or other elements of non-invasive sensor 104 can be structured to fit other appendages of a patient that are suitable for sensing hemodynamic data.

The plethysmographic signal intensity or arterial volume selected to be maintained during a volume clamp measurement made using non-invasive sensor 104 can be referred to as the plethysmographic "setpoint." This setpoint ideally corresponds to a relaxed arterial volume (i.e., undilated by arterial pulsations and unstressed or minimally-stressed by mechanical forces from air bladder 146) within the clamped volume for present conditions of the clamped volume, e.g., including patient hand position/posture and blood perfusion. In practice, the relaxed arterial volume of the appendage of patient 180 within the clamped volume can be difficult to estimate. As a result, the setpoint used for volume clamp measurements is often offset from the relaxed arterial volume, leading to potentially inaccurate arterial pressure measurements made using hemodynamic sensing system 100.

Various methods exist for re-calibrating the setpoint used for volume clamp measurements. The methods disclosed herein, conversely, provide a calibration factor that can be used to correct arterial pressure measurements made using hemodynamic sensing system 100 without requiring adjustment of the volume clamp setpoint.

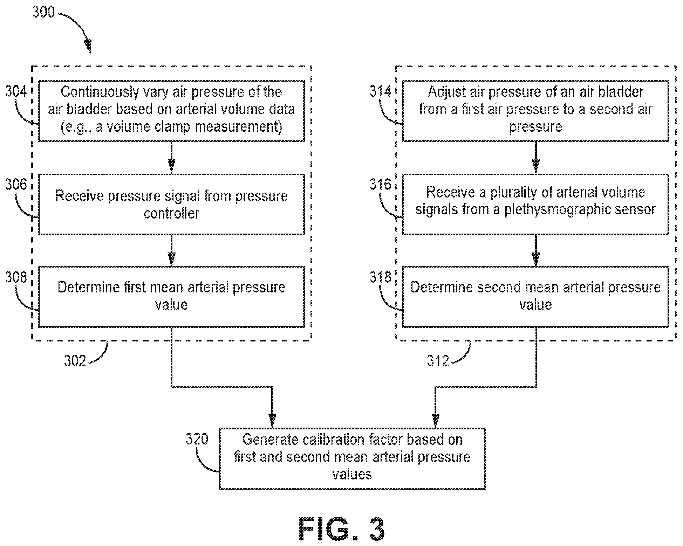

FIG. 3 is a flow diagram of method 300, which is a method of creating an arterial pressure calibration factor for calibrating arterial pressure data sensed using hemodynamic sensing system 100. Method 300 includes step set 302 (including steps 304-308) and step set 312 (including steps 314-318), which produce first and second MAP values, respectively, that are used in step 320. Step set 302 includes steps 304-308 of continuously varying air pressure of an air bladder based on arterial volume data (step 304), receiving a pressure signal from an air pressure controller (step 306), and determining a first MAP value (step 308). Step set 312 includes steps 314-318 of adjusting an air pressure of the air bladder from a first air pressure to a second pressure (step 314), receiving a plurality of arterial volume signals from a plethysmographic sensor (step 316), and determining a second MAP value (step 318). After steps of step sets 302 and 312 are performed, step 320 is performed. In step 320, a calibration factor is generated based on the first and second MAP values produced by step sets 302 and 312, respectively.

In step 304, the air pressure of air bladder 146 is varied continuously by air pressure controller 106 based on arterial volume data while air bladder 146 surrounds an appendage of patient 180 (e.g., finger 240). Processor 112 can receive plethysmographic signals representative of arterial volume of the clamped volume from plethysmographic sensor 150 and cause air pressure controller 106 to vary the pressure of air bladder 146 to maintain a plethysmographic signal setpoint.

In step 306, a pressure signal is received from an air pressure controller. As the pressure of air bladder 146 is continuously varied, air pressure controller 106 can transmit a signal to processor 112 that is representative of the pressure of air bladder 146. As described previously, this signal represents an arterial pressure waveform.

In step 308, a first MAP value is generated. If the pressure signal from the air pressure controller requires signal processing (e.g., by applying gain, noise reduction, etc.), the signal can be processed accordingly to create an arterial pressure waveform representative of arterial pressure within the clamped volume of patient 180 (i.e., the clamped volume of an appendage such as finger 240). The arterial pressure waveform can be analyzed using one or more programs of waveform processing module 130 to determine a first MAP value. For example, all or some periods of the arterial pressure waveform can be analyzed to determine average systolic and diastolic pressures, from which a MAP can be calculated. In other examples, another suitable technique for determining MAP from arterial pressure waveform data can be used. The first MAP generated in step 308 can be stored to memory 114 for use with step 320 of method 300.

During step 314, the air pressure of air bladder 146 is varied from a first pressure to a second pressure. Air pressure controller 106 adjusts the pressure of air bladder 146 to a first pressure and slowly increases the pressure of air bladder 146 according to a pressure gradient until the pressure of air bladder 146 is at the second pressure. In some examples, air pressure controller 106 can vary the pressure of air bladder 146 as a ramp. The ramp can be selected according to a ramp function that varies air pressure of the air bladder according to user preference or operational need for a given application. The ramp function can describe a ramp that is, for example, a continuous ramp or a discontinuous ramp. Where the ramp is a discontinuous ramp, the ramp function can describe a ramp that is a sequential series of steps/increments, where each step is a different pressure intermediate to the first and second pressures. Air pressure controller 106 can vary the pressure of air bladder 146 through a sequential series of steps and further can cause the pressure of air bladder 146 to be maintained at each stepped pressure value for at least one cardiac cycle (i.e., for at least one diastole and at least one systole). The type of pressure gradient used can be selected by, for example, user input at user interface 116 or by predetermined parameters stored to memory 114 (e.g., as a program or a setting of pressure control module 120), among other options. Similarly, the minimum pressure value and the maximum pressure value, as well as the length of the pressure gradient can be selected by, for example, user input at user interface 116 or can be pre-selected and stored to memory 114 (e.g., as a program or a setting of pressure control module 120), among other options. While the air pressure of air bladder 146 has been described generally herein as increasing during step 314, in other examples it may be advantageous to decrease the air pressure of air bladder 146 according to a pressure gradient and/or a ramp function.

The specific pressure values of the first pressure and second pressure of the pressure gradient can be selected according to operational need or user preference. For example, the first and second pressures can be set as rough boundaries of the expected blood pressure of a generic patient. As a specific example, the first pressure value can be set at 50mmHg (approximately 6.66612 kPa) and the second pressure value can be set at 200mmHg (approximately 26.6645 kPa). Additionally and/or alternatively, the first and second pressures can be set based on the known or expected arterial pressure of the patient. For example, if the patient has an expected arterial pressure, the first and second pressures can be percentage values (e.g., 75% and 125%, respectively) of that expected arterial pressure or can be offset by an offset amount from that expected arterial value (e.g., plus or minus 50mmHg, or another suitable offset value). In yet further examples, the first and second pressure can be selected based on the pressure data received in step 306. The first pressure can be offset from the patient's measured diastole and the second pressure can be offset from the patient's measured systole. The first pressure can be, for example, 20mmHg (approximately 2.66645 kPa) lower than patient's measured diastole and the second pressure can be, for example, 20mmHg (approximately 2.66645 kPa) higher than the patient's measured systole.

Where a pressure ramp is used, the ramp can be constrained to extend over a particular period of time. For example, the pressure gradient can be applied over a period of 20 to 30 seconds. The total time of the pressure ramp can be selected to minimize the amount of time required to perform step set 312 while allowing a sufficiently large amount of arterial volume data to be collected in subsequent step 316 to obtain a second MAP value in subsequent step 318. The total time of the pressure ramp can also be selected based on user preference, another operational need, or any other suitable parameter.

In step 316, a plurality of arterial volume signals is received from plethysmographic sensor 150. The plurality of arterial volume signals is received in step 316 while the air pressure of air bladder 146 is varied from the first arterial pressure to the second arterial pressure, such that each arterial volume signal corresponds to one pressure intermediate to the first pressure and the second pressure of the pressure gradient used in step 314. The arterial volume signals can be raw plethysmographic signals or can be processed (i.e., by application of gain, noise reduction, signal polarity inversion, etc.) prior to use in step 318.

In step 318, the plurality of arterial volume signals received in step 316 are analyzed to determine a second MAP value. Volume signals measured at air bladder 146 pressures below the MAP of arteries generally have low amplitudes, as the low pressure of air bladder 146 does not exert enough force on the arterial volume to cause the arterial volume to decrease significantly during diastole. Similarly, as the pressure of air bladder 146 is increased above the MAP, the force applied by air bladder 146 reduces the variation in volume during arterial pulsation. More specifically, at pressures above the patient's MAP, the force exerted by air bladder 146 on the clamped arterial volume can significantly reduce arterial expansion during systole, leading to waveforms with low amplitudes. Pressures near the patient's MAP apply sufficient pressure to cause arterial contraction during diastole without constricting arterial expansion during systole. Accordingly, the air pressure (i.e., the air pressure value along the gradient applied in step 314) that results in an arterial volume signal having the highest amplitude often corresponds to the MAP of arteries in the clamped volume. One or more programs of signal processing module 130 can be used to analyze the plurality of arterial volume signals received in step 316 to determine which arterial volume signal has the greatest or maximum amplitude. Processor 112 can apply a weighted means fit or a polynomial fit technique, among other options, to determine which arterial volume signal has the greatest amplitude. The pressure of air bladder 146, according to the pressure gradient used in step 314, while that arterial volume signal was measured can be stored as the second MAP.

In step 320, the second and first MAP values are used to create a calibration factor. The ratio of the second MAP value to the first MAP value can be used to scale arterial pressure waveforms obtained by a volume clamp technique. More concretely, the first and second MAP values can be used to scale arterial pressure data according to the following equation: [Equation 1] ${BP}_{adj} = \left(MAP'/MAP\right) \left({BP}_{meas}\right)$ where *BPₘₑₐₛ* is a point along the second arterial pressure waveform; *MAP'* is the second MAP value generated in step 318; *MAP* is the first MAP value generated in step 308; and *BP_{adj}* is a corresponding point along the adjusted second arterial pressure waveform. The calibration factor generated in step 320 is *MAP*'/*MAP*, and can be stored to memory 114 for application by processor 112 to any suitable arterial pressure data obtained through volume clamp measurements made with non-invasive sensor 104. Equation 1 can be applied by processor 112 to all points of an arterial pressure waveform to adjust the entire waveform. Equation 1 can be applied to already-collected data in a retroactive manner (e.g., to adjust the arterial pressure data collected in steps 304-306), or in a prospective manner to new pressure signals received from air pressure controller 106 during subsequent volume clamp measurements. In some examples, step set 312 can be performed immediately or substantially immediately after step set 302, step 320 can be performed immediately or substantially immediately after step set 312, and new volume clamp measurements can be made by varying the pressure of air bladder 146 to maintain the plethysmographic set point used in step 304 immediately after step 320 such that volume clamp measurements of the patient are interrupted only to apply the pressure gradient to the clamped volume in steps 314-316.

A MAP value generated by analyzing arterial volume data collected while air bladder 146 applies a pressure gradient (e.g., a second MAP value according to step 318) provides a more accurate estimation of patient mean arterial pressure than a MAP value generated by analyzing volume clamp data (e.g., a first MAP value according to step 308). Accordingly, MAP values produced according to step 318 can be used to calibrate pressure data generated using a volume clamp technique. As described with respect to step 320, MAP values produced according to step 318 can be combined with MAP values produced according to step 308 that can be used to scale subsequent arterial pressure data collected using a volume clamp technique.

Advantageously, method 300 allows for a calibration factor to be generated using a single hemodynamic sensor and further using the same hemodynamic sensor that is used to sense arterial pressure according to a volume clamp technique. Method 300 also allows for the accuracy of arterial pressure data collected using volume clamp techniques to be improved independently of adjustments to the volume clamp setpoint. In some examples, method 300 can be advantageously combined with methods to adjust the volume clamp setpoint to achieve even higher accuracy when measuring patient arterial pressure with a volume claim technique.

Step sets 302 and 312 can be performed in any order relative to each other. Notably, however, where a single non-invasive sensor 104 is used to perform method 300, step sets 302 and 312, and, specifically steps 304-306 and 314-316, are not performed simultaneously, as steps 304-306 require continuously varying the pressure of air bladder 146 to mechanically oppose arterial volume changes and steps 314-316 require adjusting the pressure of air bladder 146 through a pressure range while allowing arterial volume to pulsate. To this extent, steps 304-306 occur during a first time period and steps 314-316 occur during a second time period, and the first time period does not overlap the second time period.

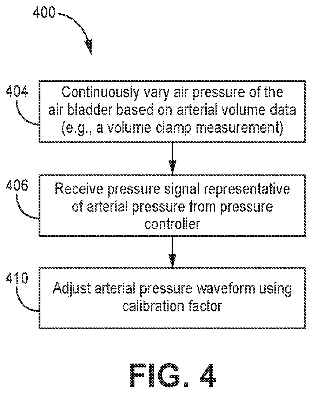

FIG. 4 is a flow diagram of method 400, which is a method of generating adjusted arterial pressure information. Method 400, and in particular step 410, can be performed following step 320 of method 300, as step 410 uses the calibration factor generated in step 320. Method 400 includes steps 304-410 of continuously varying air pressure of an air bladder based on arterial volume data (step 404), receiving a pressure signal from an air pressure controller (step 406), and adjusting an arterial pressure waveform using the calibration factor (step 410).

Steps 404 and 406 are substantially similar to steps 304 and 306 of method 300 (FIG. 3) described previously and, in some examples, are steps 304 and 306 of method 300, such that the data adjusted in step 410 is derived from the data collected in steps 304 and 306 of method 300. In other examples, the processes described with respect to steps 304 and 306 of method 300 (FIG. 3) are repeated as steps 404 and 406, respectively, to create new arterial volume data for use with method 400.

In step 410, an arterial pressure waveform is adjusted. The arterial pressure waveform is a waveform derived from the arterial pressure data received in step 306 and described the arterial pressure of arteries within the clamped volume of the patient. Each point along the arterial pressure waveform can be adjusted using the calibration factor generated in step 320 of method 300 (FIG. 3). More specifically, each point can be adjusted by multiplying each point by the calibration factor, according to equation 1 described previously. In other examples, the MAP values generated in steps 306 and 316 can be used to generate an offset that can be used to adjust measured arterial pressure values. The offset can be, for example, a linear or non-linear offset. The adjusted arterial pressure waveform generated in step 410 can be stored to memory 114 and/or output to a user via user interface 116, among other options.

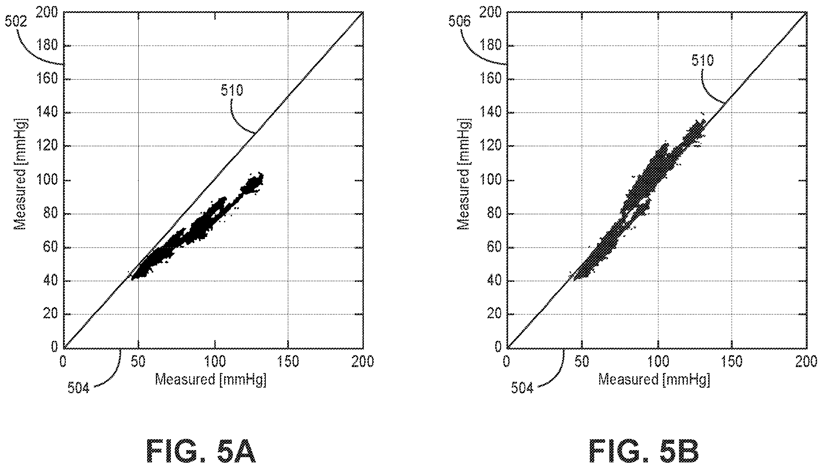

FIGS. 5A and 5B show data illustrating the ability of the calibration factors disclosed herein to improve the accuracy of blood pressure measurements made using volume clamp techniques. FIGS. 5A and 5B are scatter graphs that show various hemodynamic parameters, including diastolic pressure, systolic pressure, and MAP values, measured for a number of patients. The scatter graph shown in FIG. 5A compares clamp pressure data 502, which are values using a non-invasive volume clamping technique (e.g., according to steps 304 and 306 of method 300; FIG. 3), and catheter pressure data 504, which is measured using an invasive, catheter-based technique. FIG. 5B compares calibrated clamp pressure data 506, which is clamp pressure data 502 calibrated according to methods 300 and 400 described herein (FIGS. 3 and 4, respectively), and catheter pressure data 504. The scatter graphs shown in FIGS. 5A and 5B both include identity line 510, which indicates the value at which pressure measurements made by the techniques compared in the scatter graph are the same or have identity. As is shown in FIG. 5A, clamp pressure data 502 made using a typical volume clamp technique tends to produce lower pressure readings than catheter pressure data 504. Conversely, calibrated clamp pressure data 506 has significantly higher identity with catheter pressure data 504. Advantageously, calibrated clamp pressure data 506 was made using non-invasive techniques according to the present disclosure and did not require the invasive methods used to collect catheter pressure data 504. FIG. 5B, accordingly, illustrates the accuracy improvements that can be made by calibrating volume clamp arterial pressure data using calibration factors produced according to method 300 (FIG. 3).

The methods and systems described herein allow a single hemodynamic sensor to be used to perform arterial pressure measurements using a volume clamp technique and also to be used to create a calibration factor that can be used to improve the accuracy of arterial pressure measurements made using the hemodynamic sensor. Specifically, the methods and systems herein enable the calculation of a second MAP value using plethysmographic data taken during a pressure gradient or ramp that can be used to create a calibration factor for calibrating arterial pressure data. Further, as illustrated and discussed herein, the calibration factors described herein significantly increase the accuracy of arterial pressure measurements made using volume clamp techniques.

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc.

### DISCUSSION OF DETAILED EMBODIMENTS

The following are non-exclusive descriptions of possible embodiments of the present invention.

An embodiment of a method of measuring arterial pressure includes continuously varying an air pressure of an annular air bladder to maintain a constant volume based arterial volume data from a plethysmographic sensor, receiving a first air pressure signal representative of the air pressure of the air bladder while the air pressure is continuously varied, and determining a first mean arterial pressure value. The air pressure of the annular air bladder is continuously varied by an air pressure controller during a first time period, the first air pressure signal is received from the air pressure controller, and the first mean arterial pressure value is determined by a processor. The first air pressure signal is representative of a first arterial pressure waveform and the first mean arterial pressure value is determined based on the first arterial pressure waveform. The method further includes adjusting an air pressure of the annular air bladder from a first air pressure to a second air pressure and receiving a plurality of arterial volume signals from the plethysmographic sensor as the air pressure is adjusted from the first air pressure to the second air pressure. The air pressure is adjusted from the first pressure to the second pressure by the air pressure controller during a second time period. The plurality of arterial volume signals is received by the processor and is representative of a plurality of arterial volume waveforms. Further, each arterial volume waveform of the plurality of arterial volume waveforms corresponds to an air pressure between the first air pressure and the second air pressure. The method further comprises determining a second mean arterial pressure value based on the plurality of arterial volume waveforms and generating a calibration factor. The calibration factor is based on the second mean arterial pressure value and the first mean arterial pressure value, and is applied to arterial pressure waveform data to compensate for variations in operation of the air bladder. The second mean arterial pressure value is determined by the processor and the calibration factor is generated by the processor.

The method of measuring arterial pressure of the preceding paragraph can optionally include, additionally and/or alternatively, any one or more of the following features, configurations and/or additional components:

A further embodiment of the foregoing method of measuring arterial pressure, and further comprising continuously varying the air the air pressure of the air bladder to maintain a constant arterial volume based on arterial volume signals from the plethysmographic sensor during a third time period, receiving a second air pressure signal representative of the air pressure of the air bladder while the air pressure is continuously varied during the third time period, and generating an adjusted second arterial pressure waveform. The air pressure of the air bladder is continuously varied by the air pressure controller and the second air pressure signal is received from the air pressure controller. The adjusted second arterial pressure waveform is generated based on the calibration factor and the second arterial pressure waveform.

A further embodiment of any of the foregoing methods of measuring arterial pressure, wherein generating, by the processor, the adjusted arterial pressure waveform comprises generating the adjusted arterial pressure waveform according to the following equation: ${BP}_{adj} = \left(MAP′/MAP\right) \left({BP}_{meas}\right)$ wherein:
*BPₘₑₐₛ* is a point along the second arterial pressure waveform;
*MAP'* is the second mean arterial pressure value;
*MAP* is the first mean arterial pressure value; and
*BP_{adj}* is a corresponding point along the adjusted second arterial pressure waveform.

A further embodiment of any of the foregoing methods of measuring arterial pressure, wherein the plethysmographic sensor comprises a photoplethysmographic sensor.

A further embodiment of any of the foregoing methods of measuring arterial pressure, and further comprising analyzing, by the processor, the plurality of arterial volume waveforms with a waveform analysis technique to determine a selected arterial volume waveform of the plurality of arterial volume waveforms having a maximum amplitude, wherein determining the second mean arterial pressure value comprises determining the second mean arterial pressure value based on the pressure corresponding to the selected arterial volume waveform.

A further embodiment of any of the foregoing methods of measuring arterial pressure, wherein the waveform analysis technique comprises a weighted means fit technique.

A further embodiment of any of the foregoing methods of measuring arterial pressure, wherein the waveform analysis technique comprises a polynomial fit technique.

A further embodiment of any of the foregoing methods of measuring arterial pressure, wherein adjusting, by the air pressure controller, the air pressure of the annular air bladder from the first air pressure to the second air pressure comprises adjusting the air pressure of the annular air bladder from the first air pressure to the second air pressure according to a ramp function over a ramp period.

A further embodiment of any of the foregoing methods of measuring arterial pressure, wherein the ramp period is between 20 seconds and 30 seconds.

A further embodiment of any of the foregoing methods of measuring arterial pressure, wherein the first air pressure is 50 mmHg and the second air pressure is 200 mmHg.

A further embodiment of any of the foregoing methods of measuring arterial pressure, wherein adjusting, by the air pressure controller, the air pressure of the annular air bladder from the first air pressure to the second air pressure comprises adjusting the air pressure through a sequential series of steps of air pressures between the first air pressure and the second air pressure.

An embodiment of a system for measuring arterial pressure includes a plethysmographic sensor configured to sense arterial volume, an air pressure controller pneumatically connected to an annular air bladder and configured to adjust an air pressure of the annular airbladder, a processor in operable communication with the air pressure controller and the plethysmographic sensor, and memory encoding executable instructions. The instructions, when executed, cause the processor to cause the air pressure controller to continuously vary an air pressure of the annular air bladder to maintain a constant volume based arterial volume data from the plethysmographic sensor, receive a first air pressure signal representative of the air pressure of the air bladder while the air pressure is continuously varied, and determine a first mean arterial pressure value. The air pressure of the annular air bladder is continuously varied a first time period and the first air pressure signal is received from the air pressure controller. The first air pressure signal representative of a first arterial pressure waveform and the first mean arterial pressure value is determined based on the first arterial pressure waveform. The instructions, when executed, further cause the processor to cause the air pressure controller to adjust the air pressure of the annular air bladder from a first air pressure to a second air pressure and receive a plurality of arterial volume signals from the plethysmographic sensor as the air pressure is adjusted from the first air pressure to the air second pressure. The air pressure is adjusted from the first pressure to the second pressure by the air pressure controller during a second time period. The plurality of arterial volume signals is representative of a plurality of arterial volume waveforms and each arterial volume waveform of the plurality of arterial volume waveforms corresponds to an air pressure between the first air pressure and the second air pressure. The instructions, when executed, further cause the processor to determine a second mean arterial pressure value based on the plurality of arterial volume waveforms and generate a calibration factor. The calibration factor is based on the second mean arterial pressure value and the first mean arterial pressure value, and is applied to arterial pressure waveform data to compensate for variations in operation of the air bladder.

The system for measuring arterial pressure of the preceding paragraph can optionally include, additionally and/or alternatively, any one or more of the following features, configurations and/or additional components:
A further embodiment of the foregoing system for measuring arterial pressure, wherein the instructions, when executed, further cause the processor to cause the air pressure controller to continuously vary the air pressure of the annular air bladder to maintain a constant arterial volume based on arterial volume data from the plethysmographic sensor during a third time period, receive from the air pressure controller a second air pressure signal representative of the air pressure of the air bladder while the air pressure is continuously varied during the third time period, the second air pressure signal representative of a second arterial pressure waveform, determine a second mean arterial pressure value based on the second arterial pressure waveform, and generate an adjusted second arterial pressure waveform based on the calibration factor and the second arterial pressure waveform.

A further embodiment of any of the foregoing systems for measuring arterial pressure, wherein the instructions, when executed, further cause the processor to generate the adjusted arterial pressure waveform according to the following equation: ${BP}_{adj} = \left(MAP′/MAP\right) \left({BP}_{meas}\right)$ wherein:
*BPₘₑₐₛ* is a point along the second arterial pressure waveform;
*MAP'* is the second mean arterial pressure value;
*MAP* is the first mean arterial pressure value; and
*BP_{adj}* is a corresponding point along the adjusted second arterial pressure waveform.

A further embodiment of any of the foregoing systems for measuring arterial pressure, when executed, cause the processor to analyze the plurality of arterial volume waveforms with a waveform analysis technique to determine a selected arterial volume waveform of the plurality of arterial volume waveforms having a maximum amplitude and determine the second mean arterial pressure value based on the pressure corresponding to the selected arterial volume waveform.

A further embodiment of any of the foregoing systems for measuring arterial pressure, wherein the waveform analysis technique comprises a weighted means fit technique.

A further embodiment of any of the foregoing systems for measuring arterial pressure, wherein the waveform analysis technique comprises a polynomial fit technique.

A further embodiment of any of the foregoing systems for measuring arterial pressure, wherein the instructions, when executed, cause the processor to adjust the pressure of the air bladder from the first air pressure to the second air pressure according to a ramp function over a ramp period.

A further embodiment of any of the foregoing systems for measuring arterial pressure, wherein the ramp period is between 20 seconds and 30 seconds.

A further embodiment of any of the foregoing systems for measuring arterial pressure, wherein the first pressure is 50 mmHg and the second pressure is 200 mmHg.

A further embodiment of any of the foregoing systems for measuring arterial pressure, wherein the instructions, when executed, cause the processor to adjust the pressure of the air bladder through a sequential series of steps of air pressures between the first air pressure and the second air pressure.

A further embodiment of any of the foregoing systems for measuring arterial pressure, wherein the plethysmographic sensor comprises a photoplethysmographic sensor.

The above method(s) can be performed on a living animal or on a simulation, such as on a cadaver, cadaver heart, anthropomorphic ghost, and/or simulator (e.g., with body parts, heart, tissue, etc. being simulated).

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1. A method (300) of measuring arterial pressure, the method comprising:
continuously varying (304), by an air pressure controller and during a first time period, an air pressure of an annular air bladder to maintain a constant arterial volume based on arterial volume data from a plethysmographic sensor;
receiving (306), from the air pressure controller, a first air pressure signal representative of the air pressure of the air bladder while the air pressure is continuously varied during the first time period, the first air pressure signal representative of a first arterial pressure waveform;
determining (308), by a processor, a first mean arterial pressure value based on the first arterial pressure waveform;
adjusting (314), by the air pressure controller and during a second time period, the air pressure of the annular air bladder from a first air pressure to a second air pressure;
receiving (316), by the processor, a plurality of arterial volume signals from the plethysmographic sensor as the air pressure is adjusted from the first air pressure to the second air pressure, the plurality of arterial volume signals representative of a plurality of arterial volume waveforms and each arterial volume waveform of the plurality of arterial volume waveforms corresponding to an air pressure between the first air pressure and the second air pressure;
determining (318), by the processor, a second mean arterial pressure value based on the plurality of arterial volume waveforms; and
generating (320), by the processor, a calibration factor based on the second mean arterial pressure value and the first mean arterial pressure value, wherein the calibration factor is applied to arterial pressure waveform data to compensate for variations in operation of the air bladder.

2. The method of claim 1, and further comprising:
continuously varying (404), by the air pressure controller and during a third time period, the air pressure of the air bladder to maintain a constant arterial volume based on arterial volume signals from the plethysmographic sensor;
receiving (406), from the air pressure controller, a second air pressure signal representative of the air pressure of the air bladder while the air pressure is continuously varied during the third time period, the second air pressure signal representative of a second arterial pressure waveform; and
generating (410), by the processor, an adjusted second arterial pressure waveform based on the calibration factor and the second arterial pressure waveform.

3. The method of claim 2, wherein generating, by the processor, the adjusted arterial pressure waveform comprises generating the adjusted arterial pressure waveform according to the following equation: ${BP}_{adj} = \left(MAP′/MAP\right) \left({BP}_{meas}\right)$ wherein:
*BPₘₑₐₛ* is a point along the second arterial pressure waveform;
*MAP'* is the second mean arterial pressure value;
*MAP* is the first mean arterial pressure value; and
*BP_{adj}* is a corresponding point along the adjusted second arterial pressure waveform.

4. The method of any preceding claim, wherein the plethysmographic sensor comprises a photoplethysmographic sensor.

5. The method of any preceding claim, and further comprising analyzing, by the processor, the plurality of arterial volume waveforms with a waveform analysis technique to determine a selected arterial volume waveform of the plurality of arterial volume waveforms having a maximum amplitude, wherein determining the second mean arterial pressure value comprises determining the second mean arterial pressure value based on the pressure corresponding to the selected arterial volume waveform.

6. The method of claim 5, wherein the waveform analysis technique comprises:
a) a weighted means fit technique; or
b) a polynomial fit technique.

7. The method of any preceding claim, wherein adjusting, by the air pressure controller, the air pressure of the annular air bladder from the first air pressure to the second air pressure comprises:
a) adjusting the air pressure of the annular air bladder from the first air pressure to the second air pressure according to a ramp function over a ramp period, optionally wherein the ramp period is between 20 seconds and 30 seconds; or
b) adjusting the air pressure through a sequential series of steps of air pressures between the first air pressure and the second air pressure.

8. The method of any preceding claim, wherein the first air pressure is 50 mmHg and the second air pressure is 200 mmHg.

9. A system (100) for measuring arterial pressure, the system comprising:
a plethysmographic sensor (150) configured to sense arterial volume;
an air pressure controller (106) pneumatically connected to an annular air bladder (146) and configured to adjust an air pressure of an annular airbladder;
a processor (112) in operable communication with the air pressure controller and the plethysmographic sensor; and
a memory (114) encoding instructions that, when executed, cause the processor to:
cause the air pressure controller to continuously vary, during a first time period, the air pressure of the annular air bladder to maintain a constant arterial volume based on arterial volume data from the plethysmographic sensor;
receive, from the air pressure controller, a first air pressure signal representative of the air pressure of the air bladder while the air pressure is continuously varied during the first time period, the first air pressure signal representative of a first arterial pressure waveform;
determine a first mean arterial pressure value based on the first arterial pressure waveform;
cause the air pressure controller to adjust the air pressure of the annular airbladder from a first air pressure to a second air pressure during a second time period;
receive a plurality of arterial volume signals from the plethysmographic sensor as the air pressure is adjusted from the first air pressure to the second air pressure, the plurality of arterial volume signals representative of a plurality of arterial volume waveforms and each arterial volume waveform of the plurality of arterial volume waveforms corresponding to an air pressure between the first air pressure and the second air pressure;
determine a second mean arterial pressure value based on the plurality of arterial volume waveforms; and
generate a calibration factor based on the second mean arterial pressure value and the first mean arterial pressure value, wherein the calibration factor is applied to arterial pressure waveform data to compensate for variations in operation of the air bladder.

10. The system of claim 9, wherein the instructions, when executed, further cause the processor to:
cause the air pressure controller to continuously vary, during a third time period, the air pressure of the annular air bladder to maintain a constant arterial volume based on arterial volume data from the plethysmographic sensor;
receive, from the air pressure controller, a second air pressure signal representative of the air pressure of the air bladder while the air pressure is continuously varied during the third time period, the second air pressure signal representative of a second arterial pressure waveform;
determine a second mean arterial pressure value based on the second arterial pressure waveform; and
generate an adjusted second arterial pressure waveform based on the calibration factor and the second arterial pressure waveform.

11. The system of claim 10, wherein the instructions, when executed, further cause the processor to generate the adjusted arterial pressure waveform according to the following equation: ${BP}_{adj} = \left(MAP′/MAP\right) \left({BP}_{meas}\right)$ wherein:
*BPₘₑₐₛ* is a point along the second arterial pressure waveform;
*MAP'* is the second mean arterial pressure value;
*MAP* is the first mean arterial pressure value; and
*BP_{adj}* is a corresponding point along the adjusted second arterial pressure waveform.

12. The system of any of claims 9 to 11, wherein the instructions, when executed, cause the processor to:
analyze the plurality of arterial volume waveforms with a waveform analysis technique to determine a selected arterial volume waveform of the plurality of arterial volume waveforms having a maximum amplitude; and
determine the second mean arterial pressure value based on the pressure corresponding to the selected arterial volume waveform.

13. The system of claim 12, wherein the waveform analysis technique comprises:
a) a weighted means fit technique; or
b) a polynomial fit technique.

14. The system of any of claims 9 to 13, wherein the instructions, when executed, cause the processor to:
a) adjust the pressure of the air bladder from the first air pressure to the second air pressure according to a ramp function over a ramp period; or
b) adjust the pressure of the air bladder through a sequential series of steps of air pressures between the first air pressure and the second air pressure.

15. The system of any of claims 9 to 14, wherein the first pressure is 50 mmHg and the second pressure is 200 mmHg.

## Patentansprüche

1. Verfahren (300) zum Messen des arteriellen Drucks, wobei das Verfahren umfasst:
kontinuierliches Variieren (304) eines Luftdrucks einer ringförmigen Luftblase, um ein konstantes arterielles Volumen aufrechtzuerhalten, basierend auf arteriellen Volumendaten von einem plethysmographischen Sensor durch eine Luftdrucksteuerung und während einer ersten Zeitperiode;
Empfangen (306) eines ersten Luftdrucksignals, das für den Luftdruck der Luftblase repräsentativ ist, während der Luftdruck kontinuierlich variiert wird, während der ersten Zeitperiode von der Luftdrucksteuerung, wobei das erste Luftdrucksignal für eine erste arterielle Druckwellenform repräsentativ ist;
Bestimmen (308) eines ersten mittleren arteriellen Druckwerts basierend auf der ersten arteriellen Druckwellenform durch einen Prozessor;
Einstellen (314) des Luftdrucks der ringförmigen Luftblase von einem ersten Luftdruck auf einen zweiten Luftdruck durch die Luftdrucksteuerung und während einer zweiten Zeitperiode;
Empfangen (316) einer Vielzahl von arteriellen Volumensignalen von dem plethysmographischen Sensor durch den Prozessor, wenn der Luftdruck von dem ersten Luftdruck auf den zweiten Luftdruck eingestellt wird, wobei die Vielzahl von arteriellen Volumensignalen repräsentativ für eine Vielzahl von arteriellen Volumenwellenformen ist, und jede arterielle Volumenwellenform der Vielzahl von arteriellen Volumenwellenformen einem Luftdruck zwischen dem ersten Luftdruck und dem zweiten Luftdruck entspricht;
Bestimmen (318) eines zweiten mittleren arteriellen Druckwerts basierend auf der Vielzahl von arteriellen Volumenwellenformen durch den Prozessor; und
Generieren (320) eines Kalibrierfaktors basierend auf dem zweiten mittleren arteriellen Druckwert und dem ersten mittleren arteriellen Druckwert durch den Prozessor, wobei der Kalibrierfaktor auf arterielle Druckwellenformdaten angewendet wird, um Variationen im Betrieb der Luftblase zu kompensieren.

2. Verfahren nach Anspruch 1, das ferner umfasst:
kontinuierliches Variieren (404) des Luftdrucks der Luftblase durch die Luftdrucksteuerung und während einer dritten Zeitperiode, um ein konstantes arterielles Volumen aufrechtzuerhalten, basierend auf arteriellen Volumensignalen von dem plethysmographischen Sensor;
Empfangen (406) eines zweiten Luftdrucksignals, das für den Luftdruck der Luftblase repräsentativ ist, während der Luftdruck kontinuierlich variiert wird, während der dritten Zeitperiode von der Luftdrucksteuerung, wobei das zweite Luftdrucksignal für eine zweite arterielle Druckwellenform repräsentativ ist;
Generieren (410) einer eingestellten zweiten arteriellen Druckwellenform basierend auf dem Kalibrierfaktor und der zweiten arteriellen Druckwellenform durch den Prozessor.

3. Verfahren nach Anspruch 2, wobei Generieren der eingestellten arteriellen Druckwellenform durch den Prozessor Generieren der eingestellten arteriellen Druckwellenform gemäß der folgenden Gleichung umfasst: ${BP}_{adj} = \left(MAP′/MAP\right) \left({BP}_{meas}\right)$ wobei:
BPₘₑₐₛ ein Punkt entlang der zweiten arteriellen Druckwellenform ist;
MAP' der zweite mittlere arterielle Druckwert ist;
MAP der erste mittlere arterielle Druckwert ist; und
BP_{adj} ein entsprechender Punkt entlang der eingestellten zweiten arteriellen Druckkurve ist.

4. Verfahren nach einem der vorhergehenden Ansprüche, wobei der plethysmographische Sensor einen photoplethysmographischen Sensor umfasst.

5. Verfahren nach einem der vorhergehenden Ansprüche, das ferner Analysieren der Vielzahl von arteriellen Volumenwellenformen mit einer Wellenformanalysetechnik durch den Prozessor umfasst, um eine ausgewählte arterielle Volumenwellenform der Vielzahl von arteriellen Volumenwellenformen mit einer maximalen Amplitude zu bestimmen, wobei Bestimmen des zweiten mittleren arteriellen Druckwerts Bestimmen des zweiten mittleren arteriellen Druckwerts basierend auf dem Druck umfasst, welcher der ausgewählten arteriellen Volumenwellenform entspricht.

6. Verfahren nach Anspruch 5, wobei die Wellenformanalysetechnik umfasst:
a) eine gewichtete Mittelwertanpassungstechnik; oder
b) eine polynomiale Anpassungstechnik.

7. Verfahren nach einem der vorhergehenden Ansprüche, wobei Einstellen des Luftdrucks der ringförmigen Luftblase von dem ersten Luftdruck auf den zweiten Luftdruck durch die Luftdrucksteuerung umfasst:
a) Einstellen des Luftdrucks der ringförmigen Luftblase von dem ersten Luftdruck auf den zweiten Luftdruck gemäß einer Rampenfunktion über eine Rampenperiode, wobei die Rampenperiode gegebenenfalls zwischen 20 Sekunden und 30 Sekunden liegt; oder
b) Einstellen des Luftdrucks durch eine sequentielle Reihe von Schritten von Luftdrücken zwischen dem ersten Luftdruck und dem zweiten Luftdruck.

8. Verfahren nach einem der vorhergehenden Ansprüche, wobei der erste Luftdruck 50 mmHg beträgt und der zweite Luftdruck 200 mmHg beträgt.

9. System (100) zum Messen des arteriellen Drucks, wobei das System umfasst:
einen plethysmographischen Sensor (150), der dazu ausgelegt ist, das arterielle Volumen abzufühlen;
eine Luftdrucksteuerung (106), die pneumatisch mit einer ringförmigen Luftblase (146) verbunden ist und dazu ausgelegt ist, einen Luftdruck einer ringförmigen Luftblase einzustellen;
einen Prozessor (112) in operativer Kommunikation mit der Luftdrucksteuerung und dem plethysmographischen Sensor;
und
einen Speicher (114), der Anweisungen codiert, die bei Ausführung bewirken, dass Prozessor Folgendes ausführt:
Bewirken, dass die Luftdrucksteuerung während einer ersten Zeitperiode den Luftdruck der ringförmigen Luftblase kontinuierlich variiert, um ein konstantes arterielles Volumen aufrechtzuerhalten, basierend auf arteriellen Volumendaten von dem plethysmographischen Sensor;
Empfangen eines ersten Luftdrucksignals, das für den Luftdruck der Luftblase repräsentativ ist, während der Luftdruck kontinuierlich variiert wird, während der ersten Zeitperiode von der Luftdrucksteuerung, wobei das erste Luftdrucksignal für eine erste arterielle Druckwellenform repräsentativ ist;
Bestimmen eines ersten mittleren arteriellen Druckwerts basierend auf der ersten arteriellen Druckwellenform; und Bewirken, dass die Luftdrucksteuerung den Luftdruck der ringförmigen Luftblase von einem ersten Luftdruck auf einen zweiten Luftdruck während einer zweiten Zeitperiode einstellt;
Empfangen einer Vielzahl von arteriellen Volumensignalen von dem plethysmographischen Sensor, wenn der Luftdruck von dem ersten Luftdruck auf den zweiten Luftdruck eingestellt wird, wobei die Vielzahl von arteriellen Volumensignalen repräsentativ für eine Vielzahl von arteriellen Volumenwellenformen ist, und jede arterielle Volumenwellenform der Vielzahl von arteriellen Volumenwellenformen einem Luftdruck zwischen dem ersten Luftdruck und dem zweiten Luftdruck entspricht;
Bestimmen eines zweiten mittleren arteriellen Druckwerts basierend auf der Vielzahl von arteriellen Volumenwellenformen; und
Generieren eines Kalibrierfaktors basierend auf dem zweiten mittleren arteriellen Druckwert und dem ersten mittleren arteriellen Druckwert, wobei der Kalibrierfaktor auf arterielle Druckwellenformdaten angewendet wird, um Variationen im Betrieb der Luftblase zu kompensieren.

10. System nach Anspruch 9, wobei die Anweisungen bei Ausführung ferner bewirken, dass der Prozessor Folgendes durchführt:
Bewirken, dass die Luftdrucksteuerung während einer dritten Zeitperiode den Luftdruck der ringförmigen Luftblase kontinuierlich variiert, um ein konstantes arterielles Volumen aufrechtzuerhalten, basierend auf arteriellen Volumendaten von dem plethysmographischen Sensor;
Empfangen eines zweiten Luftdrucksignals, das für den Luftdruck der Luftblase repräsentativ ist, während der Luftdruck während der dritten Zeitperiode kontinuierlich variiert wird, von der Luftdrucksteuerung, wobei das zweite Luftdrucksignal für eine zweite arterielle Druckwellenform repräsentativ ist;
Bestimmen eines zweiten mittleren arteriellen Druckwerts basierend auf der zweiten arteriellen Druckwellenform; und
Generieren einer eingestellten zweiten arteriellen Druckwellenform basierend auf dem Kalibrierfaktor und der zweiten arteriellen Druckwellenform.

11. System nach Anspruch 10, wobei die Anweisungen bei Ausführung ferner bewirken, dass der Prozessor die eingestellte arterielle Druckwellenform gemäß der folgenden Gleichung generiert: ${BP}_{adj} = \left(MAP′/MAP\right) \left({BP}_{meas}\right)$ wobei:
BPₘₑₐₛ ein Punkt entlang der zweiten arteriellen Druckwellenform ist;
MAP' der zweite mittlere arterielle Druckwert ist;
MAP der erste mittlere arterielle Druckwert ist; und
BP_{adj} ein entsprechender Punkt entlang der eingestellten zweiten arteriellen Druckkurve ist.

12. System nach einem der Ansprüche 9 bis 11, wobei die Anweisungen bei Ausführung bewirken, dass der Prozessor Folgendes ausführt:
Analysieren der Vielzahl von arteriellen Volumenwellenformen mit einer Wellenformanalysetechnik, um eine ausgewählte arterielle Volumenwellenform der Vielzahl von arteriellen Volumenwellenformen mit einer maximalen Amplitude zu bestimmen; und
Bestimmen des zweiten mittleren arteriellen Druckwerts basierend auf dem Druck, welcher der ausgewählten arteriellen Volumenwellenform entspricht.

13. System nach Anspruch 12, wobei die Wellenformanalysetechnik umfasst:
a) eine gewichtete Mittelwertanpassungstechnik; oder
b) eine polynomiale Anpassungstechnik.

14. System nach einem der Ansprüche 9 bis 13, wobei die Anweisungen bei Ausführung bewirken, dass der Prozessor Folgendes ausführt:
a) Einstellen des Drucks der Luftblase von dem ersten Luftdruck auf den zweiten Luftdruck gemäß einer Rampenfunktion über eine Rampenperiode; oder
b) Einstellen des Drucks der Luftblase durch eine Reihe von sequentiellen Schritten von Luftdrücken zwischen dem ersten Luftdruck und dem zweiten Luftdruck.

15. System nach einem der Ansprüche 9 bis 14, wobei der erste Druck 50 mmHg beträgt und der zweite Druck 200 mmHg beträgt.

## Revendications

1. Procédé (300) de mesure de la pression artérielle, le procédé comprenant :
la variation continue (304), par un dispositif de commande de pression d'air et pendant une première période de temps, d'une pression d'air d'une poche d'air annulaire pour maintenir un volume artériel constant sur la base des données de volume artériel provenant d'un capteur pléthysmographique ;
la réception (306), en provenance du dispositif de commande de pression d'air, d'un premier signal de pression d'air représentant la pression d'air de la poche d'air pendant que la pression d'air varie en continu pendant la première période de temps, le premier signal de pression d'air représentant une première forme d'onde de pression artérielle ;
la détermination (308), par un processeur, d'une première valeur de pression artérielle moyenne sur la base de la première forme d'onde de pression artérielle ;
l'ajustement (314), par le dispositif de commande de pression d'air et pendant une deuxième période de temps, de la pression d'air de la poche d'air annulaire d'une première pression d'air à une seconde pression d'air ;
la réception (316), par le processeur, d'une pluralité de signaux de volume artériel provenant du capteur pléthysmographique lorsque la pression d'air est ajustée de la première pression d'air à la seconde pression d'air, la pluralité de signaux de volume artériel représentant une pluralité de formes d'onde de volume artériel et chaque forme d'onde de volume artériel de la pluralité de formes d'onde de volume artériel correspondant à une pression d'air entre la première et la seconde pression d'air ;
la détermination (318), par le processeur, d'une seconde valeur de pression artérielle moyenne sur la base de la pluralité de formes d'onde de volume artériel ; et
la génération (320), par le processeur, d'un facteur d'étalonnage sur la base de la seconde valeur de pression artérielle moyenne et la première valeur de pression artérielle moyenne, le facteur d'étalonnage étant appliqué aux données de forme d'onde de pression artérielle pour compenser les variations de fonctionnement de la poche d'air.

2. Procédé selon la revendication 1, et comprenant en outre :
la variation continue (404), par le dispositif de commande de pression d'air et pendant une troisième période de temps, de la pression d'air de la poche d'air afin de maintenir un volume artériel constant sur la base des signaux de volume artériel provenant du capteur pléthysmographique ;
la réception (406), en provenance du dispositif de commande de pression d'air, d'un second signal de pression d'air représentant la pression d'air de la poche d'air pendant que la pression d'air varie en continu pendant la troisième période de temps, le second signal de pression d'air représentant une seconde forme d'onde de pression artérielle ; et
la génération (410), par le processeur, d'une seconde forme d'onde de pression artérielle ajustée sur la base du facteur d'étalonnage et de la seconde forme d'onde de pression artérielle.

3. Procédé selon la revendication 2, la génération, par le processeur, de la forme d'onde de pression artérielle ajustée comprenant la génération de la forme d'onde de pression artérielle ajustée selon l'équation suivante : ${BP}_{adj} = \left(MAP′/MAP\right) \left({BP}_{meas}\right)$ avec :
BPₘₑₐₛ étant un point le long de la seconde courbe de pression artérielle ;
MAP' étant la seconde valeur de pression artérielle moyenne,
MAP étant la première valeur de pression artérielle moyenne ; et
BP_{adj} étant un point correspondant le long de la seconde courbe de pression artérielle ajustée.

4. Procédé selon l'une quelconque des revendications précédentes, le capteur pléthysmographique comprenant un capteur photopléthysmographique.

5. Procédé selon l'une quelconque des revendications précédentes, et comprenant en outre l'analyse, par le processeur, de la pluralité de formes d'onde de volume artériel avec une technique d'analyse de forme d'onde pour déterminer une forme d'onde de volume artériel sélectionnée parmi la pluralité de formes d'onde de volume artériel ayant une amplitude maximale, la détermination de la seconde valeur de pression artérielle moyenne comprenant la détermination de la seconde valeur de pression artérielle moyenne sur la base de la pression correspondant à la forme d'onde de volume artériel sélectionnée.

6. Procédé selon la revendication 5, la technique d'analyse de forme d'onde comprenant :
a) une technique d'ajustement par moyennes pondérées ; ou
b) une technique d'ajustement polynomial.

7. Procédé selon l'une quelconque des revendications précédentes, l'ajustement, par le dispositif de commande de pression d'air, de la pression d'air de la poche d'air annulaire de la première pression d'air à la seconde pression d'air comprenant :
a) l'ajustement de la pression d'air de la poche d'air annulaire d'air de la première pression d'air à la seconde pression d'air conformément à une fonction de rampe pendant une période de rampe, éventuellement la période de rampe étant comprise entre 20 secondes et 30 secondes ; ou
b) l'ajustement de la pression d'air par une série séquentielle d'étapes de pressions d'air entre la première pression d'air et la seconde pression d'air.

8. Procédé selon l'une quelconque des revendications précédentes, la première pression d'air étant de 50 mmHg et la seconde pression d'air étant de 200 mmHg.

9. Système (100) de mesure de la pression artérielle, le système comprenant :
un capteur pléthysmographique (150) configuré pour détecter un volume artériel ;
un dispositif de commande de pression d'air (106) relié pneumatiquement à une poche d'air annulaire (146) et configuré pour ajuster une pression d'air d'une poche d'air annulaire ;
un processeur (112) en communication opérationnelle avec le dispositif de commande de pression d'air et le capteur pléthysmographique ; et
une mémoire (114) encodant des instructions qui, lorsqu'elles sont exécutées, amènent le processeur à :
faire varier continuellement, pendant une première période de temps, la pression d'air de la poche d'air annulaire pour maintenir un volume artériel constant sur la base des données de volume artériel provenant du capteur pléthysmographique ;
recevoir, en provenance du dispositif de commande de pression d'air, un premier signal de pression d'air représentant la pression d'air de la poche d'air pendant que la pression d'air varie en continu pendant la première période de temps, le premier signal de pression d'air représentant une première forme d'onde de pression artérielle ;
déterminer une première valeur de pression artérielle moyenne sur la base de la première courbe de pression artérielle ;
amener le dispositif de commande de pression d'air à ajuster la pression d'air de la poche d'air annulaire d'une première pression d'air à une seconde pression d'air pendant une deuxième période de temps ;
recevoir une pluralité de signaux de volume artériel provenant du capteur pléthysmographique lorsque la pression d'air est ajustée de la première pression d'air à la seconde pression d'air, la pluralité de signaux de volume artériel représentant une pluralité de formes d'onde de volume artériel et chaque forme d'onde de volume artériel parmi la pluralité de formes d'onde de volume artériel correspondant à une pression d'air entre la première et la seconde pression d'air ;
déterminer une seconde valeur de pression artérielle moyenne sur la base de la pluralité de formes d'onde de volume artériel ; et
générer un facteur d'étalonnage sur la base de la seconde valeur de pression artérielle moyenne et de la première valeur de pression artérielle moyenne, le facteur d'étalonnage étant appliqué aux données de forme d'onde de pression artérielle pour compenser les variations de fonctionnement de la poche d'air.

10. Système selon la revendication 9, les instructions, lorsqu'elles sont exécutées, amenant en outre le processeur à :
faire varier continuellement, pendant une troisième période de temps, la pression d'air de la poche d'air annulaire pour maintenir un volume artériel constant sur la base des données de volume artériel provenant du capteur pléthysmographique ;
recevoir, en provenance du dispositif de commande de pression d'air, un second signal de pression d'air représentant la pression d'air de la poche d'air pendant que la pression d'air varie en continu pendant la troisième période de temps, le second signal de pression d'air représentant une seconde forme d'onde de pression artérielle ;
déterminer une seconde valeur de pression artérielle moyenne sur la base de la seconde courbe de pression artérielle ; et
générer une seconde courbe de pression artérielle ajustée sur la base du facteur d'étalonnage et de la seconde courbe de pression artérielle.

11. Système selon la revendication 10, les instructions, lorsqu'elles sont exécutées, amenant en outre le processeur à générer la forme d'onde de pression artérielle ajustée selon l'équation suivante : ${BP}_{adj} = \left(MAP′/MAP\right) \left({BP}_{meas}\right)$ avec :
BPₘₑₐₛ étant un point le long de la seconde courbe de pression artérielle ;
MAP' étant la seconde valeur de pression artérielle moyenne,
MAP étant la première valeur de pression artérielle moyenne ; et
BP_{adj} étant un point correspondant le long de la seconde courbe de pression artérielle ajustée.

12. Système selon l'une quelconque des revendications 9 à 11, les instructions, lorsqu'elles sont exécutées, amenant le processeur à :
analyser la pluralité de formes d'onde de volume artériel avec une technique d'analyse de forme d'onde afin de déterminer une forme d'onde de volume artériel sélectionnée de la pluralité de formes d'onde de volume artériel ayant une amplitude maximale ; et
déterminer la seconde valeur de pression artérielle moyenne sur la base de la pression correspondant à la courbe de volume artériel sélectionnée.

13. Système selon la revendication 12, la technique d'analyse de forme d'ondes comprenant :
a) une technique d'ajustement par moyennes pondérées ; ou
b) une technique d'ajustement polynomial.

14. Système selon l'une quelconque des revendications 9 à 13, les instructions, lorsqu'elles sont exécutées, amenant le processeur à :
a) ajuster la pression de la poche d'air de la première pression d'air à la seconde pression d'air conformément à une fonction de rampe pendant une période de rampe ; ou
b) ajuster la pression de la poche d'air par une série séquentielle d'étapes de pressions d'air entre la première pression d'air et la seconde pression d'air.

15. Système selon l'une quelconque des revendications 9 à 14, la première pression étant de 50 mmHg et la seconde pression étant de 200 mmHg.
